# EUROPEAN PATENT SPECIFICATION

(11) **EP 1 115 322 B1**
(45) Date of publication and mention of the grant of the patent: **13.02.2008**
(21) Application number: 99941059.0
(22) Date of filing: 11.08.1999
(51) Int. Cl.: A47L 13/16

(54) **WIPE ARTICLE HAVING A THREE-DIMENSIONAL WIPING SURFACE**
WISCHTUCH MIT EINER DREIDIMENSIONALEN WISCHFLÄCHE
ARTICLE DE NETTOYAGE, PRESENTANT UNE SURFACE DE NETTOYAGE TRIDIMENSIONNELLE

(30) Priority: 12.08.1998 US 132833
(43) Date of publication of application: 18.07.2001
(73) Proprietor: THE PROCTER & GAMBLE COMPANY, Cincinnati, Ohio 45202 (US)
(72) Inventor: HANSER, Thomas, Robert, Taylor Mill, KY 41015 (US); GORLEY, Ronald, Thomas, Cincinnati, OH 45241 (US); TOUSSANT, John, William, West Chester, OH 45069 (US)
(74) Representative: Kremer, Véronique Marie Joséphine
(86) International application number: PCT/US1999/018254
(87) International publication number: WO 2000/008998

(56) References cited:
- WO-A-98/52458
- US-A- 3 965 518
- US-A- 4 144 370
- US-A- 4 683 001

## Description

### FIELD OF THE INVENTION

The present invention is related to disposable wiping articles, and more particularly to disposable wiping articles having a macroscopically three dimensional wiping surface.

### BACKGROUND OF THE INVENTION

Disposable wiping articles are well known in the art. Such wiping articles typically have a substrate which includes one or more materials or layers. The substrate can be pre-moistened with a wetting agent prior to use, or alternatively, can be combined with a liquid at the point of use of the article. Pre-moistened wiping articles are also referred to as "wet wipes" and "towelettes." Efforts have been made to increase the surface texture of wet wipes to provide a relatively soft wiping surface that can provide effective, yet gentle cleaning.

US-A-3 965 518 describes a cleaning article consisting of nonwoven cellulosic paper material web, whereby the substrate is made soft by creping and whereto bounding material is applied according to a particular pattern forming an embossed layer.

US-A-4 144 370 describes a fabric comprising a base layer which is an open woven fabric forming open areas comprising high entangled fibres.

Wipe substrates generally comprise nonwoven materials, including meltblown, spunbonded, and carded webs. Nonwoven substrates are often embossed to add a certain amount of texture to a wipe article produced from the nonwoven. However, embossing does not add significant bulk, or caliper to the nonwoven web, so that the increase in texture is negligible.

Other methods of imparting texture to a nonwoven web, or a composite web comprising, for example, an elastic film and a nonwoven, are known. Such methods include stretch bonding elastic materials to non-elastic materials to form stretchable composites by methods that result in a web having a plurality of generally parallel gathers, wrinkles or rugosities. However, the resulting repeating, nonrandom pattern of surface texture can be perceived as more harsh than a random, nonrepeating pattern.

In addition to producing nonrandom, repeating patterns, known methods of imparting texture to webs suitable for wipe substrates, such as by stretch bonding, do not impart a random, nonrepeating pattern of surface texture having significant Z-direction (the direction through the thickness of material) caliper increase. A significant increase would result in a macroscopically three dimensional surface that is perceived as soft, and has significantly increased surface area for improved cleaning of soil. In particular, a wet wipe having a three dimensional surface would have improved ability to clean a baby of fecal material during diaper changes.

Accordingly, it would be desirable to provide a disposable wiping article having a macroscopically three dimensional surface which exhibits texture and bulk for improved wiping.

Also, it would be desirable to provide a disposable wiping article having a macroscopically three dimensional surface exhibiting a random, nonrepeating texture.

Also, it would be desirable to provide a disposable wet wipe, such as a disposable wet wipe for babies, which has an elastically-extensible macroscopically three-dimensional surface for improved cleaning of fecal material.

Further, it would be desirable to provide an improved premoistened wipe which can be packaged for use as a wipe for cleaning fecal material from infants or incontinent adults.

### SUMMARY OF THE INVENTION

A disposable wiping article is disclosed. The disposable wiping article has a macroscopically three dimensional surface and comprises an elastic web material and at least one nonwoven web joined to the elastic web at least at two areas, the nonwoven web being gathered between the two areas. The nonwoven is elastic. The nonwoven web is a first layer, intermittently joined to the elastic web material in a face to face relationship, which is a second layer.

Portions of the first layer are gathered by contraction of the second layer relative to the first layer, thereby providing the macroscopically three dimensional surface of the first layer. The three dimensional surface of the first layer has relatively elevated peaks and relatively depressed valleys. The peaks of the first layer provide elongated, elevated ridges.

The resulting soft, deformable ridges are believed to provide a relatively soft wiping surface as compared to embossed surfaces. As a result, the wiping article of the present invention can provide effective, yet gentle cleaning.

Further, without being limited by theory, it is believed that the wiping article of the present invention avoids repeating, nonrandom surface texture, which can be perceived as more harsh than a random, nonrepeating pattern.

The macroscopically three dimensional surface is characterized by the Average Height Differential between the peaks and the valleys, the Average Peak to Peak Distance, and the nondimensional Surface Topography Index, which is the ratio of the Average Height Differential to the Average Peak to Peak Distance. The Average Height Differential can be at least about 0.5 mm, more preferably at least about 1.0 mm, and still more preferably at least about 1.5 mm. The Average Peak to Peak Distance can be at least about 1.0 mm, more preferably at least about 1.5 mm, and still more preferably at least about 2.0 mm. In one embodiment, the Average Peak to Peak distance is between about 2 to 20 mm, and more particularly, between about 4 to 12 mm. The Surface Topography Index can be at least 0.10, and less than about 2.5. In one embodiment, the Surface Topography Index is at least about 0.10, preferably about 0.15, and more preferably at least about 0.20.

Preferably, the disposable wiping article includes a third layer, wherein the second layer is disposed between the first layer and the third layer. The third layer can be of substantially the same form as the first layer, or alternatively, can be different from the first layer. In one embodiment, the first and third layers are nonwoven webs of substantially the same material and construction, and each of the first and third layers is gathered by contraction of the second layer to provide elongated ridges on the outwardly facing surfaces of each of the first and third layers.

### BRIEF DESCRIPTION OF THE DRAWINGS

Figure 1 is a plan view schematic illustration of a three layer embodiment of a wiping article of the present invention, wherein the second layer comprises a scrim material having filaments which run parallel to the side and end edges of the article, wherein a portion of the first layer is shown cut away, and wherein surface features of the first layer are omitted for clarity.
Figure 2 is an illustration of a wiping article of the type shown in Figure 1 depicting an alternative embodiment of the present invention wherein the filaments of the second layer are inclined at an angle of about 45 degrees relative to the side and end edges of the article.
Figure 3 is a plan view schematic illustration showing the texture of the macroscopically three-dimensional outer surface of the first layer, and particularly the extended ridges on the outer surface of the first layer, of the wiping article of the type shown in Figure 1.
Figure 4 is a cross-sectional illustration of an article of the type shown in Figure 1, taken parallel to one of the filaments of the second layer and showing portions of the filament extending intermediate the filament intersections, the portions of the filament being unbonded to the first layer, as well as portions of the filaments extending intermediate the filament intersections which are unbonded to the third layer.
Figure 5 is a photomicrograph showing the texture of the macroscopically three dimensional surface of the first layer of an article of the type shown in Figure 1, and in particular the elongated ridges of the surface. The scale in Figure 5 is in inches.
Figure 6 is a enlarged photomicrograph of the surface shown in Figure 5 showing an elongated ridge having branches extending in different directions.
Figure 7 is a Scanning Electron Micrograph providing a perspective view of the macroscopically three dimensional surface of the first layer of an article of the type shown in Figure 1.
Figure 8 is a Scanning Electron Micrograph of a cross-section of an article of the type shown in Figure 1 showing portions of filaments extending intermediate filament intersections, which portions of the filaments are unbonded to the first layer.
Figure 9 is a Scanning Electron Micrograph of an article of the type shown in Figure 1 showing bonding of the first and third layers to the second layer at the filament intersections.
Figure 10 is a plan view schematic illustration of a three layer embodiment of a wiping article of the present invention, wherein the second layer comprises a composite formed of two scrim layers overlaid to form a scrim having generally rectangular openings of varying sizes, wherein a portion of the first and second layers are shown cut away, and wherein surface features of the first layer are omitted for clarity.
Figure 11 is a plan view schematic illustration of a three layer embodiment of a wiping article of the present invention, wherein the second layer comprises a composite formed of two scrim layers overlaid to form a scrim having a plurality of generally triangular openings of varying sizes, wherein a portion of the first and second layers are shown cut away, and wherein surface features of the first layer are omitted for clarity.
Figure 12 is a plan view schematic illustration of a three layer embodiment of a wiping article of the present invention, wherein the second layer comprises a film material, wherein a portion of the first layer is shown cut away, and wherein surface features of the first layer are omitted for clarity.
Figure 13 is an illustration of a wiping article of the type shown in Figure 10 depicting an alternative embodiment of the present invention wherein the film material of the second layer is an apertured formed film.
Figure 14 is a schematic illustration of an apparatus for making the wiping article of the type shown in Figures 12 and 13.

### DETAILED DESCRIPTION OF THE INVENTION

As used herein, the term "macroscopically three dimensional" means a three dimensional structure or pattern which is readily visible to the naked eye when the perpendicular distance between the viewer's eye and the plane of the article being viewed is about 30 cm (12 inches). In other words, the three-dimensional structures of the present invention are cleaning sheets that are nonplanar, in that one or both surfaces of the sheet exist in multiple planes, where the difference in elevation between those planes is observable to the normal, naked eye when the structure is observed from about 30 cm (12 inches). By way of contrast, the term "planar" refers to cleaning sheets having fine-scale surface aberrations on one or both sides, the surface aberrations not being readily visible to the naked eye when the perpendicular distance between the viewer's eye and the plane of the web is about 30 cm (12 inches) or greater. In other words, on a macroscale, the observer would not observe that one or both surfaces of the sheet exist in multiple planes so as to be three-dimensional.

The term "elastic" is used herein to mean any material which, upon application of a biasing force, is stretchable, that is, elongatable, to a stretched, biased length which is at least about 125 percent, that is about one and one quarter, of its relaxed, unbiased length, and which, will recover at least 40 percent of its elongation upon release of the stretching, elongating force. A hypothetical example that would satisfy this definition of an elastic material would be a 2.5 cm (one (1) inch) sample of a material which is elongatable to at least 3.2 cm (1.25 inches) and which, upon being elongated to 3.2 cm (1.25 inches) and released, will recover to a length of not more than 2.9 cm (1.15 inches) Many elastic materials may be stretched by much more than 25 percent of their relaxed length, for example, 100 percent or more, and many of these will recover to substantially their original relaxed length, for example, to within 105 percent of their original relaxed length, upon release of the stretching, elongating force.

Many materials, particularly nonwoven materials, may be elastic in one direction. For example, the material may be elastic in the cross-machine direction, or the machine direction, or both directions. In each case, the material is considered elastic.

Many materials have variable elastic properties based upon the number of cycles of elongation/release which they experience. For the purposes of the present invention, it is only required that a material experience elastic recovery for one cycle.

Many materials will have variable elastic properties based upon the amount of time elongated, and the amount of time after release the material is allowed to recover. In a wet wipes context, the time of elongation and recovery are typically short, on the order of several seconds. Therefore, without being bound by theory, the time of elongation and the time for recovery is generally preferred to be within about 10-60 seconds.

As used herein, the term "non-elastic" refers to any material which does not fall within the definition of "elastic," above.

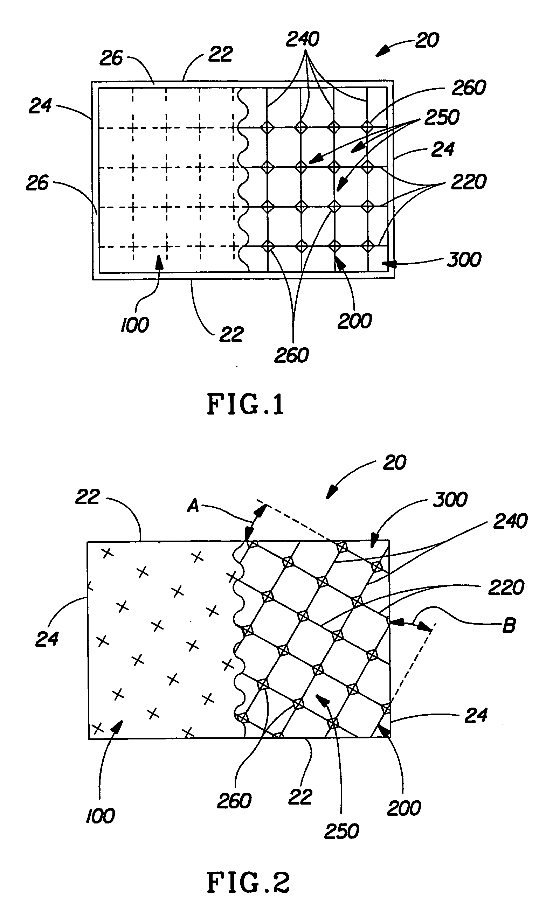

As used herein, the term "recover" refers to a contraction of a stretched material upon termination of a biasing force following stretching of the material by application of the biasing force. For example, if a material having a relaxed, unbiased length of 2.5 cm (one (1) inch) is elongated 50 percent by stretching to a length 3.8 cm of (one and one half (1.5) inches) the material would be elongated 50 percent and would have a stretched length that is 150 percent of its relaxed length of its relaxed length. If this exemplary stretched material contracted, that is recovered to a length of 2.7 cm (one and one tenth (1.1) inches) after release of the biasing and stretching force, the material would have recovered 80 percent 1 cm ((0.4) cm inch) of its elongation. Figure 1 illustrates one embodiment of a multiple layer disposable wiping article **20** according to the present invention. The wiping article **20** includes side edges **22** and end edges **24.** The side edges **22** extend generally parallel to the length of the article **20,** and the end edges **24** extend generally parallel to the width of the article. Optionally, the article **20** can include an edge seal **26** extending around the perimeter of the article. Such an edge seal **26** can be formed by heating, by use of adhesives, or by a combination of heating and adhesives.

The wiping article **20** includes a first layer **100** and a second layer **200.** Preferably, the wiping article also includes a third layer **300.** The second layer **200** can be disposed between the first layer **100** and the third layer **300.** In Figure 1, a portion of the first layer **100** is shown cut away to reveal underlying portions of the second layer **200** and the third layer **300.**

The first layer **100** is formed from nonwoven materials, such as are known in the art. Particularly preferred materials are nonwoven webs having fibers or filaments distributed randomly as in "air-laying" or certain "wet-laying" processes, or with a degree of orientation, as in certain "wet-laying" and "carding" processes. The fibers or filaments of the first layer **100** can be natural, or of natural origin (e.g. cellulosic fibers such as wood pulp fibers, cotton linters, rayon, and bagasse fibers) or synthetic (e.g. polyolefins, polyamides or polyesters), or blends thereof. Basis weights can range from about 15 grams per square metre (gsm) up to about 75 (gsm.) In a preferred embodiment the basis weight is from about 18 (gsm) to about 33 (gsm). In a more preferred embodiment the basis weight is from about 20 (gsm) to about 25 (gsm). The third layer **300** can be substantially the same as the first layer **100,** or alternatively, can be of a different material and/or construction.

In one embodiment, the first layer **100** and the third layer **300** can each comprise a hydroentangled web of synthetic nonwoven fibers having a denier of less than about 4.0, preferably less than about 3.0, and more preferably less than about 2.0 grams per 9000 meter of fiber length. A suitable first layer **100** (as well as a suitable third layer **300)** is a hydroentangled web of polyester fibers having a denier of about 1.5 grams per 9000 meters of fiber length or less, and the web having a basis weight of about 30 grams per square meter. A suitable web is available from PGI Nonwovens of Benson, N.C. under the designation PGI 9936.

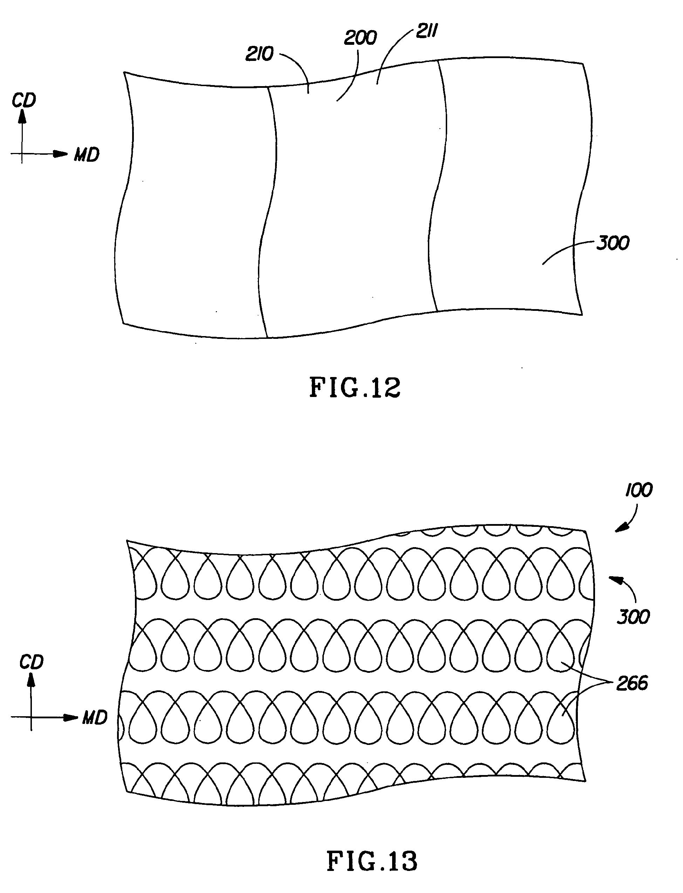

The second layer **200** is joined in a discontinuous manner to the first layer **100,** and provides gathering of the first layer, such as by contraction of the second layer **200** relative to the first layer **100** when the layers are heated and subsequently cooled. The second layer **200** can have openings therethrough. In one embodiment, the second layer **200** comprises a net-like arrangement of filaments having openings defined by adjacent filaments. Alternatively, the second layer could comprise an apertured layer having openings therethrough, or an embossed layer having surface depressions instead of or in addition to openings. For instance, the second layer **200** could be an apertured, embossed or planar (i.e., non-apertured, non-embossed, fluid impervious) plastic film, as described more fully below with reference to Figure 12.

In the embodiments illustrated in Figures 1-9, the second layer comprises a net like arrangement of filaments including a first plurality of filaments **220** and a second plurality of filaments **240.** The filaments **220** extend generally parallel to one another, and the filaments **240** extend generally parallel to one another and generally perpendicular to the filaments **220.** The filaments extend between filament intersections **260.** The intersecting, adjacent filaments **220** and **240** define openings **250** in the second layer **200.** The filament intersections and openings **250** are arranged in a generally nonrandom, repeating grid-like pattern. In one embodiment, the scrim layer can be in a non-uniform pattern, that is, the grid can comprise filaments having unequal filament-to-filament spacing.

The second layer **200** can comprise a polymeric net (referred to herein as a "scrim material"). Suitable scrim materials are described in U.S. Patent 4,636,419. The scrim may be derived from a polyolefin such as polyethylene or polypropylene, copolymers thereof, poly(butylene terephthalate), polyethylene terephthalate, ethylene vinyl acetate, Nylon 6, Nylon 66, and the like.

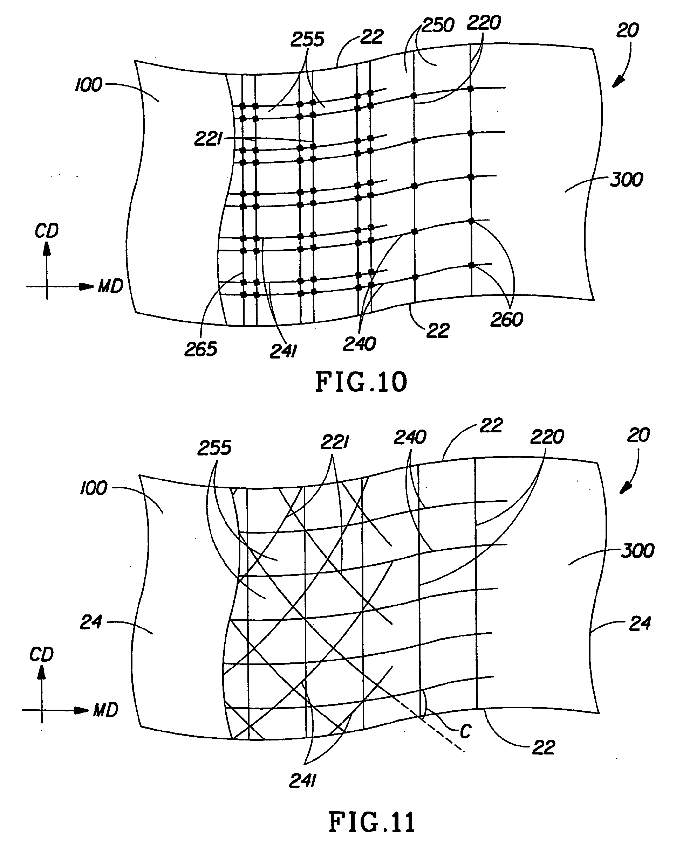

The second layer **200** can also comprise multiple layers of scrim materials to form a composite scrim **265** of offset grid patterns. For example, two scrim layers can be superimposed, with grid patterns offset such that the resulting composite scrim **265** comprises non-uniform openings **255,** as shown in Figure 10. In addition to the filaments **220** and **240,** a second scrim material can have a plurality of generally parallel filaments **221,** also being generally parallel to filaments **220** of the first scrim material. Likewise, generally parallel filaments **241** of the second scrim material can be generally parallel to filaments **240** of the first scrim material. The non-uniform openings can result in a more randomized, non-repeating pattern of the nonwoven of the resulting wiping article.

While two scrim materials having similar filament to filament spacings are shown in Figure 10, such a configuration is meant to be illustrative, and not limiting. Scrim layers having filaments on varying filament to filament spacing can be used. Additionally, more than two scrims may be used to produce a composite scrim having numerous variations on filament to filament spacing, and opening **255** sizes. In one embodiment two overlapping scrims are used as illustrated in Figure 10, with one scrim having a repeat pattern with twice the spacing of the other and aligned to form concentric squares.

In general, it is preferable that the scrim materials be joined one to the other such that the composite scrim produced handles as a single scrim during processing and use of a wet wipe. Joining can be by melt bonding filaments of one scrim to filaments of another scrim at substantially all the cross over points. For practical purposes, a single scrim having the pattern of two or more scrims overlaid would suffice to produce much of the benefit of multiple scrims. Without being bound by theory, however, there may be some added benefit to having two overlaid scrims, particularly if there is not complete bonding between the scrim materials, such that contraction is not uniform across the web.

In another embodiment, two scrim materials may be joined into a composite scrim to form non-rectangular openings **255.** For example, as shown in Figure 11, filaments **221** of second scrim material can be oriented at an angle C to filaments **220,** and filaments **241** of the second scrim material can be oriented at a similar angle to filaments **240** of first scrim material. In this manner, non-rectangular shaped openings **255** can be formed, such as the numerous substantially triangular shaped openings **255** shown in Figure 11. A single scrim having tri-axially oriented filaments, such that the openings are substantially triangular would serve a substantially similar purpose, and could eliminate the need to join two scrim materials.

The scrim material is joined to the layers **100** and **300** through lamination via heat or chemical means such as adhesives. Preferably, the filaments of the scrim material contract relative to the layers **100** and **300** upon heating and subsequent cooling, such that contraction of the second layer **200** gathers the layers **100** and **300,** and imparts a macroscopic three dimensional texture to the outer surfaces of the layers **100** and **300,** as is described in more detail below.

In another embodiment, the scrim materials could be dissimilar materials, for example, a "weak" material joined to a "strong" material. That is, the filaments of one layer may have different contraction characteristics, such that the amount of contraction differs, for example in the machine direction versus the cross direction.

A particularly suitable scrim material useful as the second layer **200** is a heat activated reinforcing netting available from Conwed Plastics of Minneapolis, MN as THERMANET brand reinforcing netting, Number R05060 having a polypropylene/EVA resin, two-sided adhesive, and a filament count of 3 filaments per 2.5 cm (inch) by 2 filaments per 2.5 cm (inch) prior to contraction such as by heating. After heating, the second layer 200 can have between about 3.5 to 4.5 filaments per 2.5 cm (inch) by between about 2.5 to 3.5 filaments per 2.5 cm (inch).

By "two-sided adhesive" it is meant that the EVA adhesive (Ethyl-Vinyl Acetate adhesive) is present on both sides of the filaments. The activation temperature of the EVA is generally about 85 Centigrade (about 185 Fahrenheit). During lamination of the layer **200** to the polyester fibers of the layers **100** and **300,** the EVA adhesive is activated to provide bonding between the filaments of the layer **200** and the fibers of the layers **100** and **300.** Without being limited by theory, it is believed that pressing at a relatively low pressure (e.g. less than 345 kilopascals (50 psi) and more preferably less than 172 kilopascals (25 psi) for a relatively short time (e.g. less than about 30 seconds), the filaments of the layer 200 are not continuously bonded to the nonwovens of layers **100** and **300.** This discontinuous bonding, along with the shrinkage of the polypropylene filaments upon heating, provides enhanced texture of the outward surfaces of layers **100** and **300.**

In Figure 1, the filaments **220** extend generally parallel to the side edges **22** and to the length of the article **20.** Likewise, the filaments **240** extend generally parallel to the end edges **24** and to the width of the article **20.**

Alternatively, the filaments **220** can be inclined at an angle of between about **20** and about 70 degrees with respect to the length of the article **20** and the side edges **22,** and more preferably between about 30 degrees and about 60 degrees. The filaments **240** can be inclined at an angle of between about 20 and about 70 degrees with respect to the width of the article **20** and the end edges **24,** and more preferably between about 30 degrees and about 60 degrees.

Figure 2 shows an embodiment of the present invention wherein the filaments **220** are inclined at an angle of about 45 degrees with respect to the side edges **22** (Angle A in Figure 2), and wherein the filaments **240** are inclined at an angle of about 45 degrees with respect to the end edges **24** (Angle B in Figure 2). Such an arrangement provides the advantage that the angled orientation of the filaments **220** and **240** with respect to the length and width of the article **20** permits deformation of the net structure of layer **200** parallel to the edges **22** and **24.** Such deformation provides the article with elastic like behavior parallel to the length and width of the article.

By "elastic like behavior" parallel to a direction of the article it is meant that the article can be elongated under tension in that direction to have an elongated dimension measured in that direction which is at least 120 percent of the article's original, relaxed dimension in that direction, and that upon release of the elongating tension the article recovers to within 10 percent of its relaxed dimension.

An important aspect of the present invention is that the first layer **100** is intermittently bonded to the second layer **200.** In particular, the first layer **100** can be intermittently bonded to the second layer **200** at the filament intersections **260,** while portions of the filaments **220,** portions of the filaments **240,** or portions of both the filaments **220** and **240** intermediate the filament intersections **260** remain unbonded to the first layer **100.**

As a result, the surface texture of the outer surface of the first layer **100** is not limited by the geometry of the openings in the net-like arrangement of filaments, but rather, is decoupled from the repeating, nonrandom geometry of the openings **250.** Similarly, the third layer **300** can be intermittently bonded to the second layer **200** to provide similar surface texture to the outer surface of the third layer **300.**

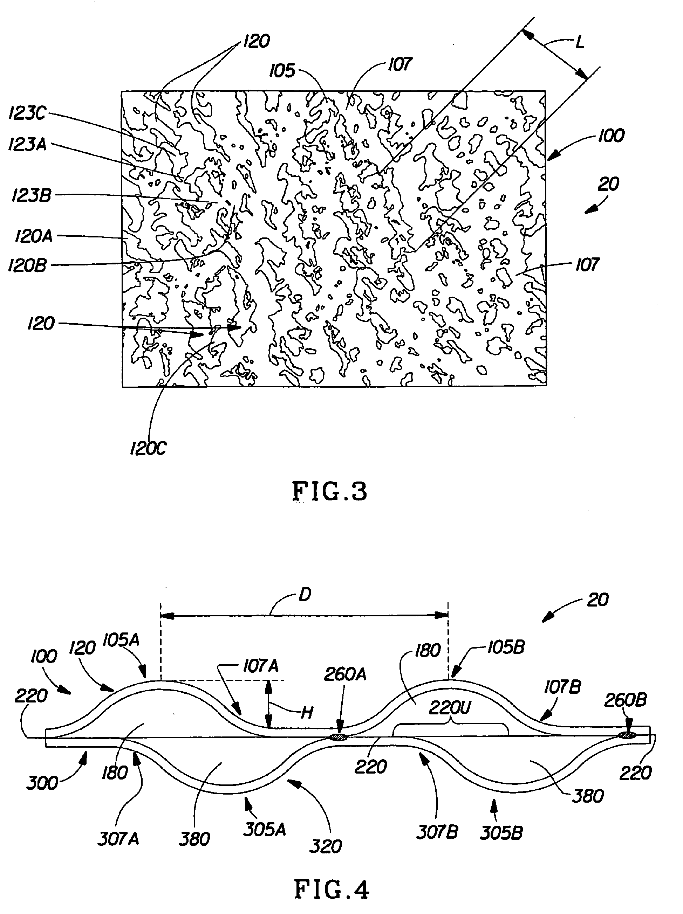

The surface texture of the first layer **100** is omitted in Figures 1 and 2 for clarity. The surface texture is shown in Figures 3-8.

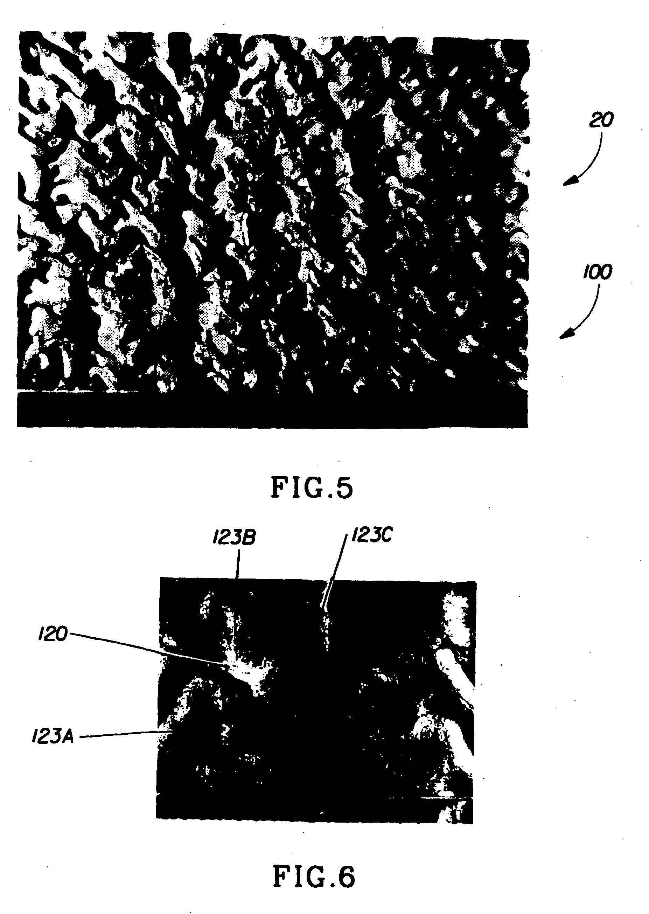

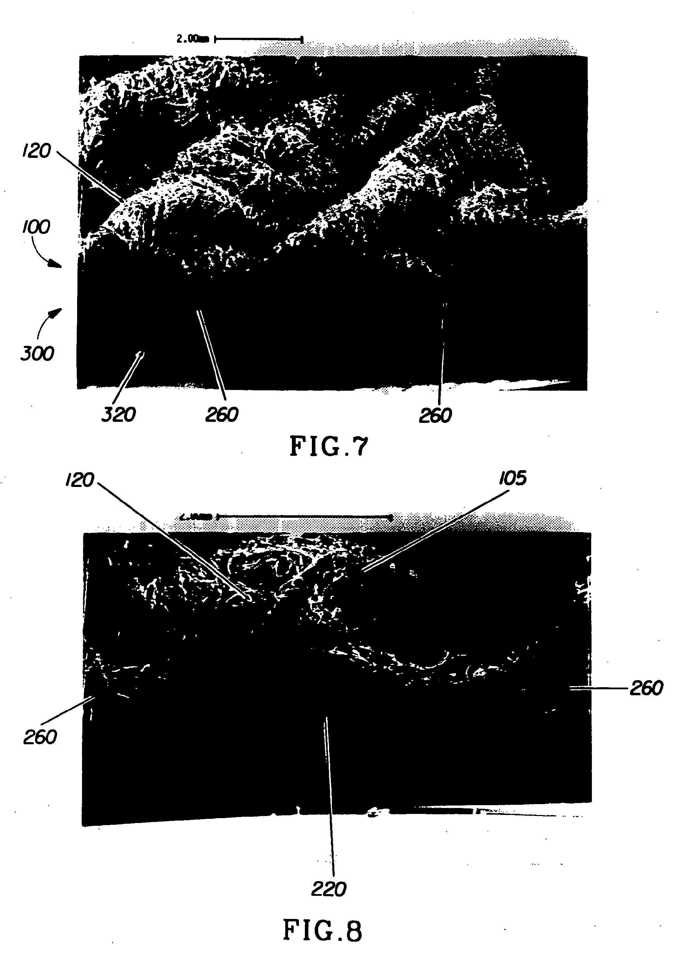

Figure 3 provides a schematic illustration of the surface texture of first layer **100** shown in the photograph of Figure 5. Figure 4 provides a cross-sectional illustration of the surface texture of the first layer **100** and the third layer **300.** Figure 5 is a photomicrograph showing the texture of the macroscopically three dimensional surface of the first layer **100.** Figure 6 is a photomicrograph showing the three dimensional surface of the first layer **100** enlarged. Figure 7 is a scanning electron micrograph providing a perspective view of the three dimensional surface of the first layer **100.** Figure 8 is a scanning electron micrograph of a cross-section of the article.

Referring to Figure 3-8, portions of the first layer **100** are gathered by contraction of the second layer **200** relative to the first layer **100.** This gathering provides the first layer **100** with a macroscopically three dimensional surface as illustrated in Figure 3-8. Likewise, the third layer **300** can be gathered by contraction of the second layer **200** to provide the third layer **300** with a macroscopically three dimensional surface.

The three dimensional surface of the first layer **100** has relatively elevated peaks **105** and relatively depressed valleys **107.** The third layer has peaks **305** and valleys **307.** In Figure 4, the peaks of layer **100** are indicated with reference numbers **105A** and **105B,** and the valleys of layer **100** are indicated with reference numbers **107A** and **107B.** Similarly, the peaks of layer **300** are labeled **305A** and **305B,** and the valleys are labeled **307A** and **307B.** The peaks **105** provide elongated ridges **120** on the outward surface of the first layer **100,** and the peaks **305** provide elongated ridges **320** on the outward surface of the third layer **300.**

The macroscopic three dimensionality of the outer surface of the first layer **100** can be described in terms of the "Average Height Differential" of a peak and an adjacent valley, as well as in terms of the "Average Peak-to-Peak Distance" between adjacent peaks. The height differential with respect to a peak **105A/**valley **107A** pair is the distance **H** in Figure 4. The peak-to-peak distance between an adjacent pair of peaks **105A** and **105B** is indicated as distance **D** in Figure 4. The "Average Height Differential" and the "Average Peak-to-Peak Distance" for the article are measured as set forth below in "Test Methods." The "Surface Topography Index" of the outward surface is the ratio obtained by dividing the Average Height Differential of the surface by the Average Peak to Peak Distance of the surface.

Without being limited by theory, it is believed that the Surface Topography Index is a measure of the effectiveness of the macroscopically three dimensional surface in receiving and containing material in the valleys of the surface. A relatively high value of Average Height Differential for a given Average Peak to Peak Distance provides deep, narrow valleys which can trap and hold materials. In particular, such an arrangement is desirable for receiving and containing fecal material. Accordingly, a relatively high value of Surface Topography Index is believed to indicate effective capture of materials during wiping.

The Average Height Differential of the outward surface of the first layer **100** and the third layer **300** can be at least about 0.5 mm, more preferably at least about 1.0 mm, and still more preferably at least about 1.5 mm. The Average Peak to Peak Distance can be at least about 1.0 mm, more preferably at least about 1.5 mm, and still more preferably at least about 2.0 mm. In one embodiment, the Average Peak to Peak distance is between about 2.0 to 20 mm, and more particularly, between about 4.0 to 12 mm. The Surface Topography Index can be at least 0.10, and less than about 2.5. In one embodiment, the Surface Topography Index is at least about 0.10, and more preferably at least about 0.20.

The wiping articles of the present invention have the characteristic that portions of the filaments **220,** portions of the filaments **240,** or portions of both the filaments **220** and **240** of the second layer **200** are not bonded to the first layer **100.** Referring to Figure 4, a portion of a filament **220** extending intermediate filament intersections **260A** and **260B** is not bonded to the first layer **100.** The portion of the filament **220** which is not bonded to the first layer **100** is indicated by reference number **220U.** A gap between the filament **220** and the first layer **100** provides a void space **180** intermediate the first layer **100** and the filament **220.** Similarly, portions of the filament **220** extending intermediate filament intersections **260** are not bonded to the third layer **300,** thereby providing a void space **380** intermediate the third layer **300** and the filament **220.**

Figures 7 and 8 also illustrate this characteristic of the article **20.** In Figure 7, elongated ridges **120** and **320** are visible on the outward surfaces of both the first and third layers **100, 300,** respectively. In Figure 8, a filament **220** is seen extending between two filament intersections **260.** The portion of the filament extending between the two filament intersections is spaced from, and not bonded to, the first layer.

Ridges **120** are shown in plan view in Figure 3 and Figure 5. At least some of the ridges **120** extend across at least one filament of the second layer **200.** In Figure 4, the ridge **120** corresponding to peak **105A** extends across at least one filament **220.**

Because the ridges extend across one or more filaments, the ridges can have a length greater than the maximum distance between adjacent filament intersections **260** (the distance between adjacent filament intersections after contraction of layer **200** and gathering of layers **100** and **300**)**.** In particular, the length of the ridges **120** can be greater than the maximum dimension of the openings **250** in Figure 1 (i.e. greater than the length of the diagonal extending across the rectangular openings **250**)**.** The length of a ridge **120** is indicated by the letter L in Figure 3. The Length L is the straight line distance between two ends of a ridge **120,** the ends of the ridge **120** being those points where a ridge **120** terminates at a valley **107.**

The value of L can be at least about 1.0 centimeter, more particularly at least about 1.5 centimeter for some of the ridges **120.** In one embodiment, at least some of the ridges **120** have a length L of at least about 2.0 centimeters. The length L can be at least twice the distance between adjacent filament intersections.

For instance, in order to determine the length of ridges **120** relative to the distance between adjacent filament intersections, the wiping article **20** can be wetted (if not premoistened) and positioned on a light table or other suitable source of back lighting. Such back lighting, in combination with wetting of the wiping article, can be used to make the filament intersections of the layer **200** visible through the layer **100,** so that the lengths of ridges **120** relative to the distance between filament intersections can be measured with a scale.

The elongated ridges provide soft, deformable wiping elements for enhanced removal of material from the surface being cleaned. In contrast, if the filaments of the second layer were continuously bonded to the first and second layers, then any texture features of the first and third layers would be confined to the area associated with the openings **250** in the second layer **200.**

At least some of the elongated ridges extend in a direction different from at least some of the other ridges. Referring to Figure 3, the ridges **120A, 120B,** and **120C** each extend in a different direction. Accordingly, the article is effective to pick up material when the article is used to wipe in different directions.

Figures 3 and 6 also illustrate that at least some of the ridges **120** can have branches extending in different directions. In Figure 3, a ridge **120** is shown having three branches **123A, 123B,** and **123C** extending in different directions. Likewise, Figure 6 shows a ridge **120** having at least three branches labeled **123A, 123B,** and **123C.**

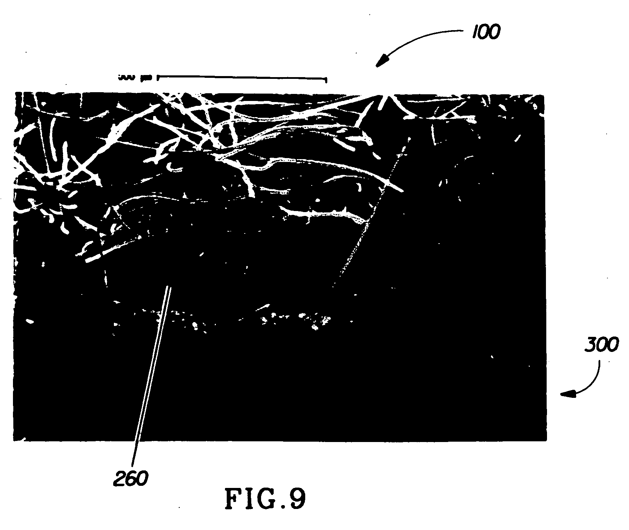

The first layer **100** and the third layer **300** are securely bonded to the second layer **200** at the filament intersections **260.** Figure 9 illustrates the bonding of fibers of both the layers **100** and **300** to the second layer at a filament intersection **260.**

Referring to Figures 4, 7 and 8, the peaks **105** of the first layer **100** are generally offset from the peaks **305** of the third layer in the plane of the article **20.** For instance, in Figure 4 the peak **305A** of the third layer does not directly underlie the peak **105A,** but instead is generally aligned with the valley **107A** associated with peak **105A.** Accordingly, the peaks **105** of the first layer are generally aligned with valleys **307** of the third layer, and the peaks **305** of the third layer are generally aligned with valleys **107** of the first layer.

The present invention also includes a method for making a multiple layer wiping article having a second scrim layer. A first nonwoven layer, a second scrim layer comprising a net like arrangement of filaments, and a third nonwoven layer are provided. The first layer is positioned adjacent an upper surface of the second layer, in face to face relationship with the second layer. The third layer is positioned adjacent a lower surface of the second layer, in face to face relationship with the second layer.

The first layer and the third layer are then intermittently bonded to discrete, spaced apart portions of the second layer, such that portions of the filaments extending between filament intersections remain unbonded to the first layer, and such that portions of the filaments extending between filament intersections remain unbonded to the third layer. The second layer is contracted relative to the first layer and the third layer to provide a gathered, macroscopically three dimensional outward surface of the first layer, and a gathered, macroscopically three dimensional outward surface of the third layer. The steps of bonding and contracting can occur simultaneously, or in sequence.

The step of intermittently bonding the second layer to the first layer and the third layer can comprise the step of heated pressing of the first layer, the second layer, and third layer at a relatively low pressure for a relatively short time period to avoid relatively continuous bonding of the second layer to the first and third layers.

In one embodiment, the three layers can be joined using a BASIX B400 hand press manufactured by the HIX Corp. of Pittsburg, Kansas. The three layers are joined by pressing in the hand press at a temperature of about 166°C (330 Fahrenheit) for about 13 seconds. The hand press has an adjustment for varying the clearance, and hence the pressure, provided in the press. The adjustment can be varied as desired to provide the desired texture in the layers **100** and **300.**

In Figure 12, another embodiment of wipe of the present invention is shown. In Figure 12, a portion of the first layer **100** is shown cut away to reveal underlying portions of the second layer **200,** which is likewise cutaway to reveal underlying portions of third layer **300.** In the embodiment shown, a polymeric film **210** is used as the second layer **200** intermittently bonded to first layer **100** and third layer **300,** to form a three layer web. It has been found that use of a polymeric film can impart the desired random, non-repeating pattern of macroscopic three dimensional texture to the outer surfaces of the layers **100** and **300,** while providing for other processing and use benefits. Gathering and contraction of first and third layers forms the macroscopic three dimensional surface texture, similar to that shown in Figures 3-8. The surface texture of the first and third layers is omitted in Figure 12 for clarity.

Polymeric film **200** is preferably a planar (i.e., non-apertured, non-embossed) film. Use of a planar film provides for a moisture impervious barrier within each wet wipe. A moisture impervious barrier prevents or significantly delays the migration of moisture to the bottom of the dispensing tub during long periods of being stacked prior to use. Therefore, wet wipes having a predetermined amount of moisture when produced into a stack and placed in a tub for dispensing tend to stay moist longer.

While a planar film is preferred for polymeric film **200,** other films can be used with other benefits. For example, a vacuum formed or hydroformed three dimensional apertured film can provide added caliper to the finished wipe. The apertures formed by vacuum forming or hydroforming typically have tapered capillaries that provide for preferential fluid flow, which can aid in pulling soil particles into the central portion of the wet wipe during use. Suitable formed polymeric films 200 may be made according to the teachings of any of commonly assigned U.S. Pat. Nos. 4,342,314 issued to Radel et al. on Aug. 3, 1982; 4,609,518 issued to Curro et al. on Sep. 2, 1986; and 4,778,644 issued to Curro et al. On Oct. 18, 1988.

Polymeric film **210** may be intermittently bonded to first layer **100** and third layer **300** and heat-contracted to form a macroscopically three-dimensional surface on the first and third layers. Intermittent bonding can be accomplished by the application of a suitable adhesive to the nonwoven layers in one or more spiral patterns, for example as shown in Figure 13, prior to bonding and application of heat to contract second layer **200.** Other bonding methods, including meltblowing adhesive, may be used to achieve the desired randomness of the final nonwoven texture.

The spiral pattern shown in Figure 13 is representative of a preferred method of spiral bond glue application. In Figure 13, five spirals of glue have been applied to a nonwoven web (**100** or **300**) prior to bonding to second layer **200.** The number and size of glue spirals **266** impacts the resulting pattern of texture in the finished wipe, and may be varied to obtain the desired surface topography characteristics of the finished wipe. It is believed that by providing for some amount of overlap between adjacent spirals, as shown in Figure 13, a more random, nonrepeating pattern is produced in the finished wipe. In another embodiment the glue is applied in a meltblown method that provides for a more controlled application rate. In general, an optimum glue application rate to each nonwoven is 3 mg per 6.45 cm² (square inch). The glue rate can be as high as 7 mg per square inch (10.85 gsm) grams per square metre.

In a preferred embodiment, a general construction hot melt adhesive, such as Ato Findley 2545, available from Ato Findley of Wauwatosa, WI is used, applied at a rate of preferably about 3.5 mg per square inch to each nonwoven, in a pattern of meltblown hot melt adhesive fibers in a three-fourths inch pattern per nozzle. Glue may be applied by a melt blown gluing system as supplied by J&M Laboratories, Inc. of Dqwsonville, GA and wequipped with DF2 nozzles capable of being operated to produce three-fourths inch glue patterns of the specified weight per nozzle by methods known in the art.

Suitable polymeric films **210** may be intermittently bonded to first and third layers and heat contracted in a similar manner that disclosed for the scrim materials described above. However, in a preferred embodiment, polymeric film **210** is an elastic film **211** intermittently bonded to first and third layers while in an elastically extended state, and thereafter allowed to elastically contract. Bonding is accomplished by applying a glue pattern to the nonwoven layers, as described with reference to Figure 13, above. Once bonded intermittently to elastic film **211,** the first and third nonwoven layers gather and contract upon release of the tension of second layer **200.**

In a one embodiment, elastic film **211** comprises a 0.5 mil thick linear low density polyethylene film such as Exxon EMV685. In another embodiment elastic film **211** comprises a 0.5 thick blend of metallocene linear low density polyethylene and low density polyethylene diamond micropettern embossed film such as Exxon EMB-685. Other elastomeric films such as polyolefin elastomers (e.g., INSIGHT® or ENGAGE® polymers from Dow Chemical, Midland MI.), polyester elastomers (e.g., HYTREL® and blends thereof), and EVA films may also be used as the second layer of a wipe of the present invention.

First and (if used) third layers may be elastic nonwoven webs having suitable softness and texture for use as a wet wipe. In one embodiment, the nonwoven web comprises 100% polyester spunlaced nonwoven having a basis weight of 33 gsm, such as PGI #9936 available from PGI Nonwovens of Landisville NJ. This nonwoven has elastic properties such that after being stretched to 125% of its original length in the cross machine direction and released, it recovers to 109% of its original length. In another embodiment, the nonwoven web comprises a through air thermal bonded carded blend of 40% polypropylene, 40% polyethylene, and 20% rayon, having a basis weight of 25 gsm, obtained from PGI as PGI-98-016. This nonwoven has elastic properties such that after being stretched to 125% of its original length in the cross machine direction and released, it recovers to 103% of its original length. In another embodiment, the nonwoven web comprises a calender thermal bonded carded blend of 55% polypropylene, 25% polyethylene, and 20% rayon, having a basis weight of 20 gsm, obtained from Fiber Visions Inc. as HER-98-003. This nonwoven has elastic properties such that after being stretched to 125% of its original length in the cross machine direction and released, it recovers to 103% of its original length.

Patterns of embossing may be imparted by calendaring processes on nonwoven webs used for first and third layers **(100** and **300,** respectively), however, it is believed that minimal embossing is desirable for maximum softness. In one embodiment, the nonwoven web is thermally bonded by through-air bonding, without any embossing. Through-air bonded nonwovens are generally lower density, which translates to caliper and softness increase in the finished wipe.

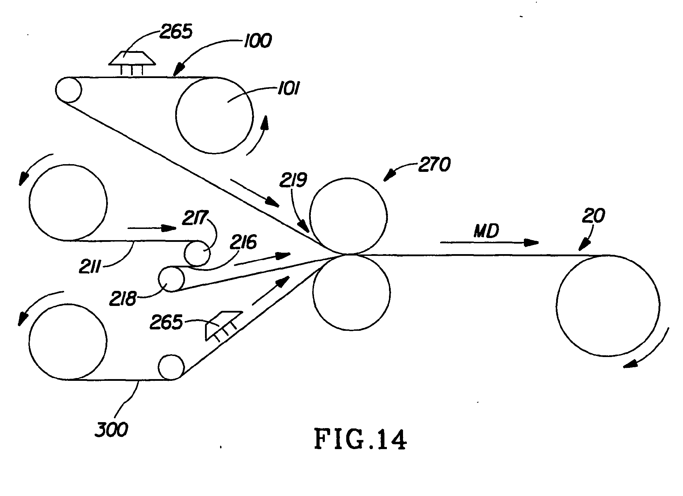

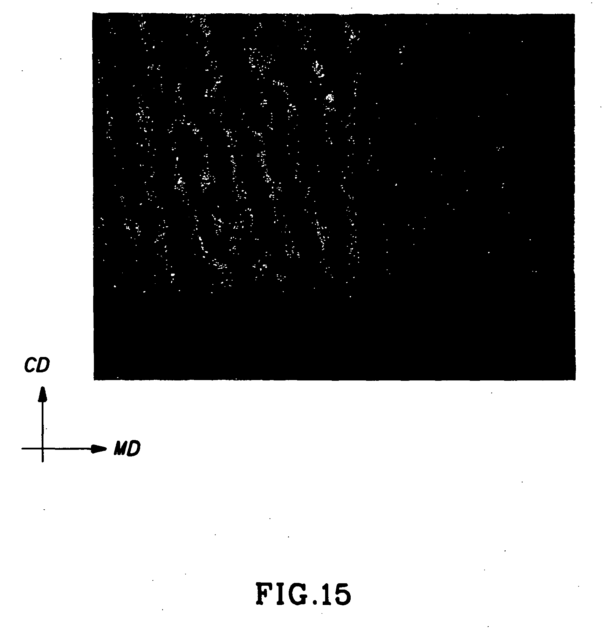

The three layer wipe shown in Figure 12 having an elastic polymeric layer **211** may be produced by the method and apparatus shown schematically in Figure 14. The elastic web **211** may be unwound from a supply roll **201** of the elastic web material. The web **211** then travels in the direction indicated by the arrows associated therewith and passes through the nip **216** formed by the stacked rollers **217** and **218.** While stacked rollers are shown, it is apparent that any form of nip rollers sufficient to apply tension to elastic web **211** may be employed with equivalent results. From the stacked rollers the web passes through the pressure nip **219** formed by a bonder roller arrangement **270** which serves as to effect pressure bonding of the layers as disclosed more fully below.

A first nonwoven web **100** is unwound from a supply roll **101** and a second nonwoven web **300** is unwound from a supply roll **301.** The nonwoven webs **100** and **300** travel in the directions indicated by the arrows associated respectively therewith as supply rolls **101** and **301** rotate in the directions indicated by the respective arrows associated therewith.

Each nonwoven web can have adhesive applied by glue applicators **265.** Glue applicators **265** may apply glue in spiral patterns, meltblown patterns, or other patterns that provide for intermittent bonding of the nonwoven layers to second layer **200.**

After application of glue, nonwoven webs **100** and **300** are directed to pass through the pressure nip **219** of the bonder roller arrangement **270** on the two opposite sides of the elastic web **200.** By virtue of the fact that the peripheral linear speed of the rollers **217** and **218** of the stacked rolls arrangement is controlled to be less than the peripheral linear speed of the rollers of the bonder roll arrangement **270,** the elastic web **200** is stretched to a selected percent elongation and maintained in such stretched condition during bonding of the nonwoven webs **100** and **300** to elastic web **200** during their passage through the bonder roller arrangement **270.** Bonding is effected by pressure of the component layers. Pressure need only be sufficient to effect intermittent bonding. The level of pressure can be varied to produce the desired level of bonding, and therefore, the desired surface topology of the finished web.

In one embodiment, the peripheral speed of the rollers of the bonder roll arrangement **270** is from about 20% to about 40% faster, and preferably about 25% to about 30% faster, than supply rolls **101** and **301.** A preferred three dimensional texture of the surface of the finished wipe can be achieved by maintaining a certain percent stretch that depends upon the combined basis weight of the nonwoven webs. Without being bound by theory, it is believed that a ratio of percent stretch to the combined basis weights of the nonwoven plies in gsm should be less than 0.8, preferably less than 0.6, and more preferably less than 0.5 (with all ratio units %/gsm).

In a preferred embodiment, first and third layers (**100** and **300,** respectively) comprise elastic nonwoven webs. Elastic nonwoven webs are provide the benefits of increased wiping effectiveness, increased entrapment of soil particles such as fecal material, and increased softness. Without being bound by theory, it is believed that as the wipe is used against the skin, the three dimensional surface deforms elastically, allowing increased surface area to be in contact with the skin. Once the wiping action is complete, the elastically-exposed surface area recovers back to a pre-wipe configuration, entrapping soil within the folds and gathers of the three-dimensional surface of the wipe.

The wiping article **20** can be impregnated with a liquid composition to provide a premoistened wipe, or "wet wipe." The liquid composition can be water based (at least 50 percent by weight water), and can include a number of ingredients in addition to water, including but not limited to preservatives, surfactants, emollients, moisturizers (including but not limited to humectants and skin conditioning agents), fragrances, and fragrance solubilizers, as well as other ingredients. The liquid composition is preferably at least 85 percent by weight water. The dry substrate comprising the three layers **100, 200, 300** can be saturated with about 1.5 grams to about 4.5 grams of the liquid composition per gram of the dry substrate, and in one embodiment, between about 2.0 and 3.0 grams of liquid composition per gram of dry substrate.

Preferably, the wiping article **200** is premoistened with a liquid composition comprising at least 85 percent by weight water and an effective amount of a surfactant, an effective amount of an emollient, an effective amount of preservative, an effective amount of a humectant, an effective amount of a fragrance, and an effective amount of a fragrance solubilizer.

The described embodiment of the wiping article of the present invention provides the advantage that even when wetted with a liquid composition to provide a premoistened wipe, the wiping article can maintain a macroscopically three dimensional surface having the desired Average Height Differential, Average Peak to Peak Distance, and Surface Topography Index.

In one embodiment, the liquid composition includes at least about 95 percent by weight water. The liquid composition can also include about 0.5-5.0 percent by weight Propylene Glycol, which can serve as an emollient and humectant; about 0.1-3.0 percent by weight PEG-75 Lanolin, which can serve as an emollient; about 0.1-3 percent by weight Cocoamphodiacetate, which can serve as a surfactant for cleansing the skin; about 0.1-3 percent by weight Polysorbate 20, which can serve as a surfactant for cleansing the skin and as an emulsifier for solubilizing fragrance components; about 0.01-0.3 percent by weight Methylparaben, which can serve as a preservative; about 0.005-0.10 percent by weight Propylparaben, which can serve as a preservative; about 0.005-0.1 percent by weight 2-Bromo-2-Nitropropane-1, 3-Diol, which can serve as a preservative; and about 0.02-1.0 percent by weight of a fragrance component.

In another embodiment, the liquid composition can include at least about 95 percent by weight water, about 0.01-1 percent by weight Tetrasodium EDTA, about 0.05-0.8 percent by weight Potassium Sorbate, about 0.1-5.0 percent by weight Propylene Glycol, about 0.1-3.0 percent by weight PEG 75 Lanolin, about 0.1-3 percent by weight C12-13 Pareth-7, about 0.1-2.0 percent by weight Polysorbate 20; about 0.01-1.0 percent by weight Disodium Phosphate, about 0.10-1.0 percent by weight Phenoxyethanol, about 0.01-0.5 percent by weight Benzalkonium Chloride; about 0.01-1.0 percent by weight Citric Acid, and about 0.02-1.0 percent by weight of a fragrance component.

Other liquid compositions with which the substrate can be moistened are described in the following patent documents U.S. Patent 4,941,995 issued July 17, 1990 to Richards et al.; U.S. Patent 4,904,524 issued February 27, 1990 to Yoh; U.S. Patent 4,772,501 issued September 20, 1988 to Johnson et al.

According to the present invention, the disposable wiping article can be premoistened, with a plurality of premoistened wipes being packaged in a suitable package. A suitable package can include a tub type container, such as is disclosed in U.S. Patent 5,065,887 issued November 19, 1991 to Schuh et al. The tub container can be wrapped in a generally moisture impervious wrap, such as a heat shrinkable polymeric film.

The package can include instructions related to using the premoistened wiping article for cleaning body parts, including removal of fecal material from the skin. The instructions can include a description of wiping with the wiping article, including wiping in different directions in order to take advantage of the different orientation of the ridges, as well as initial stretching of the wiping article followed by contraction of the article to trap the materials in the valleys of the surfaces of the wiping article.

### TEST METHODS:

In measuring the Average Peak to Peak Distance and the Average Height Differential, the following procedure is used. The method can be used to measure samples that are dry, samples that are premoistened (wet), and/or samples that have dried out (e.g. premoistened samples that have been dried).

Prior to taking measurements, a straight guide line is drawn on the surface of interest (e.g. the outward surface of layer **100**) using a permanent extra fine marker, such as a Sharpie brand extra fine point permanent marker. The guide line is drawn taking care not to distort the surface being measured. The guide line can serve as a focusing aid in making measurements. As an additional aid, "ridge lines" can also be drawn along the ridge peaks, the "ridge lines" intersecting the guide line to facilitate measurement of the peak spacing.

### Average Peak to Peak Distance:

Simple light microscopy is used to measure the distance between adjacent peaks located along the guide line. A simple clear plastic ruler having appropriate markings (e.g., millimeter markings) may be used to aid in measuring the peak to peak distance. In the alternative, a stereoscope equipped with measurement capability (e.g. Optimus version 4.2) may be used. The peak to peak distance is the shortest distance between each pair of adjacent peaks located along the guide line. If the ridges are perpendicular to the guide line, then the peak to peak distance is measured along the guide line. If the ridges are not perpendicular to the guide line, the peak to peak distance is measured along a direction which intersects the guide line midway between the two adjacent peaks and which provides the shortest distance between the two adjacent peaks. At least 10 such measurements are taken. The Average Peak to Peak Distance is the average of these measurements.

### Average Height Differential:

The Average Height Differential is determined using a light microscope (e.g. Zeiss Axioplan, Zeiss Company Germany) equipped with a depth measuring device (e.g. Microcode II, sold by Boeckeler Instruments) which provides a reading related to a change in height for a given change in focus of the microscope.

Measurements of height differential are taken along the same portion of the guide line from which the Peak to Peak measurements are taken. The microscope is focused on a peak along the guide line, and the depth measuring device is zeroed. The microscope is then moved to an adjacent valley along the guide line, and the microscope is refocused on the surface of the valley along the guide line. The display of the depth measuring device indicates the relative height difference between the peak/valley pair (the distance H in Figure 4). In general, two height measurements will be obtained for each peak to peak distance measurement, corresponding to the descent from a peak to a valley and the ascent from the valley to the adjacent peak. This measurement is repeated for the peak/valley pairs encountered along the guide line. The Average Height Differential is the average of these measurements.

The Average Peak to Peak Distance and the Average Height Differential can be calculated based on measurements made along any convenient guide line provided at least 10 consecutive peak pairs can be identified without encountering edge effects or other abnormalities.

### Surface Topography Index:

The Surface Topography Index is the ratio of Average Peak to Peak Distance and the Average Peak to Valley Height Differential. In a preferred embodiment, the Surface Topography Index is at least about 0.15, and more preferably about 0.20.

### Example 1:

A wiping article **20** according to the present invention includes a first layer **100,** a second layer **200,** and a third layer **300.** The first layer 100 and the third layer **300** each comprise a spunlace elastic nonwoven web of polyester fibers having a basis weight of about 33 grams per square meter and having the properties of after being stretched to 125% of its original length, returns to 109% upon removal of the stretching forces. The second layer comprises the above described EXXON 0.5 mil thick blend of Metallocene linear low density polyethylene and low density polyethylene diamond micropattern embossed film Number Exxon EMB-685. The second layer **200** is positioned between the first layer **100** and the third layer **300** and stretchably attached as hereinabove described.

The wiping article has the measured values of peak to peak distance and height differential listed in Table I. Table I also lists the Average Peak to Peak Distance, the Average Height Differential, and the Surface Topography Index for the sample. In this particular example, the sample was measured in its wet state.

**TABLE I**

| WET | | |
|---|---|---|
| **Adjacent Peak Pair Number** | **Peak to Peak Distance (mm)** | **Peak/Valley Height Differential (mm)** |
| | | |
| 1 | 4.2 | 0.7 |
| | | 1.0 |
| 2 | 3.7 | 1.0 |
| | | 0.6 |
| 3 | 5.8 | 0.4 |
| | | 1.2 |
| 4 | 3.8 | 1.2 |
| | | 1.0 |
| 5 | 5.3 | 1.1 |
| | | 0.5 |
| 6 | 3.4 | 0.8 |
| | | 1.5 |
| 7 | 5.3 | 1.0 |
| | | 0.0 |
| 8 | 4.9 | 0.9 |
| | | 1.5 |
| 9 | 4.9 | 1.0 |
| | | 0.9 |
| 10 | 4.0 | 1.8 |
| | | 1.5 |
| AVERAGE | 4.5 mm | 0.98 mm |
| **Surface Topography Index** | | **0.22** |

### Comparative Example:

By way of example, a commercial baby wipe product, "HUGGIES® Supreme Care" baby wipes was characterized using the test methods described above. This wiping article is sold in a package marked with the U.S. Pat. No. 4,741,944. Two sample were measured for peak to peak distance and height differential, with the data listed in Table II below. Table II also lists the Average Peak to Peak Distance, the Average Height Differential, and the Surface Topography Index for two samples tested. In this particular example, the two HUGGIES® Supreme Care wipe samples were measured in their as-purchased wet state.

**TABLE II**

| | Sample 1 | | Sample 2 | |
|---|---|---|---|---|
| | WET as PURCHASED | | WET as PURCHASED | |
| **Adjacent Peak-Pair Number** | **Peak to Peak Distance (mm)** | **Peak/Valley Height Differential (mm)** | **Peak to Peak Distance (mm)** | **Peak/Valley Height Differential (mm)** |
| | | | | |
| 1 | 3.2 | 0.6 | 3.0 | 0.4 |
| | | 0.3 | | 0.5 |
| 2 | 2.9 | 0.4 | 3.0 | 0.2 |
| | | 0.2 | | 0.1 |
| 3 | 3.8 | 0.2 | 3.6 | 0.3 |
| | | 0.5 | | 0.3 |
| 4 | 2.5 | 0.4 | 2.7 | 0.5 |
| | | 0.4 | | 0.3 |
| 5 | 3.7 | 0.4 | 2.4 | 0.1 |
| | | 0.3 | | 0.1 |
| 6 | 2.9 | 0.4 | 1.9 | 0.3 |
| | | 0.6 | | 0.3 |
| 7 | 3.4 | 0.6 | 2.9 | 0.1 |
| | | 0.5 | | 0.2 |
| 8 | 3.6 | 0.5 | 3.8 | 0.4 |
| | | 0.3 | | 0.3 |
| 9 | 3.6 | 0.4 | 3.1 | 0.1 |
| | | 0.5 | | 0.1 |
| 10 | 2.5 | 0.4 | 3.8 | 0.3 |
| | | 0.5 | | 0.1 |
| AVERAGE | 3.2 mm | 0.4 mm | 3.0 mm | 0.3 mm |
| **Surface Topography Index** | | **0.13** | | **0.1** |

A wiping article having the recited values of Surface Topography, Average Peak to Peak Distance, and/or Average Height Differential, as claimed, and as calculated from measurements made in a dry, wet, or dried state along any selected guide line, is considered to be within the scope of the present invention.

## Claims

1. A disposable wiping article (20), said disposable wiping article (20) comprising:
a) an elastic web material (200); and
b) at least one elastic nonwoven web (100),
wherein said at least one elastic nonwoven web (100) is joined to said elastic web (200) in a face to face relationship in at least two areas, and whereby said at least one elastic nonwoven web (100) is gathered between said at least two areas by contraction of said elastic web material (200) providing a macroscopically three dimensional surface of said at least one elastic nonwoven web (100) and wherein both said elastic web material (200) and said at least one elastic nonwoven web (100) are stretchable to at least 125% of their relaxed length and recover at least 40% of their elongation upon release of the elongating force.

2. The disposable wiping article (20) of Claim 1 wherein said elastic web material (200) comprises a polymeric material chosen from the group consisting of polyolefins such as polyethylene or polypropylene, polyolefin elastomers, polyesters, polyester elastomers, poly(butylene terephthalate), polyethylene terephthalate, ethylene vinyl acetate or nylon, and blends and copolymers thereof.

3. The disposable wiping article (20) of Claim 1, wherein said at least one elastic nonwoven (100) comprises fibres that are natural or synthetic or blends thereof.

4. The disposable wiping article (20) of Claim 1, wherein said at least one elastic nonwoven (100) further comprises an outward, macroscopically three dimensional surface having a random, non-repeating arrangement of peaks and valleys, wherein the macroscopically three dimensional surface has a Surface Topography Index of at least about 0.15.

5. The disposable wiping article (20) of Claim 4, wherein said macroscopically three dimensional surface has a Surface Topography Index of at least about 0.20.

6. The disposable wiping article (20) of Claim 4, wherein said macroscopically three dimensional surface has a Average Height Differential of at least about 0.5 mm.

7. The disposable wiping article (20) of Claim 4, wherein said macroscopically three dimensional surface has an Average Peak to Peak Distance of at least about 2.0 mm.

## Patentansprüche

1. Einwegwischartikel (20), wobei der Einwegwischartikel Folgendes umfasst:
(a) ein elastisches Bahnenmaterial (200); und
(b) mindestens eine elastische Vliesbahn (100),
wobei die mindestens eine elastische Vliesbahn (100) in mindestens zwei Bereichen Fläche auf Fläche an die elastischen Bahn (200) angefügt ist und wobei die mindestens eine elastische Vliesbahn (100) zwischen den mindestens zwei Bereichen durch Zusammenziehen des elastischen Bahnenmaterials (200) gerafft ist, wodurch eine makroskopisch dreidimensionale Oberfläche der mindestens einen elastischen Vliesbahn (100) bereitgestellt wird und wobei sowohl das elastische Bahnenmaterial (200) als auch die mindestens eine elastische Vliesbahn (100) auf mindestens 125 % ihrer entspannten Länge dehnbar sind und sich beim Loslassen der Dehnungskraft zu mindestens 40 % ihrer Verlängerung erholen.

2. Einwegwischartikel (20) nach Anspruch 1, wobei das elastische Bahnenmaterial (200) ein Polymermaterial umfasst, das ausgewählt ist aus der Gruppe, bestehend aus Polyolefinen, wie Polyethylen oder Polypropylen, Polyolefinelastomeren, Polyestern, Polyesterelastomeren, Poly(butylenterephthalat), Polyethylenterephthalat, Ethylenvinylacetat oder Nylon und Mischungen und Copolymeren davon.

3. Einwegwischartikel (20) nach Anspruch 1, wobei der mindestens eine elastische Vliesstoff (100) Fasern umfasst, die natürlich oder synthetisch oder Mischungen davon sind.

4. Einwegwischartikel (20) nach Anspruch 1, wobei der mindestens eine elastische Vliesstoff (100) ferner eine äußere, makroskopisch dreidimensionale Oberfläche mit einer zufälligen, nichtwiederholenden Anordnung von Erhebungen und Vertiefungen umfasst, wobei die makroskopisch dreidimensionale Oberfläche einen Oberflächentopographieindex von mindestens ungefähr 0,15 aufweist.

5. Einwegwischartikel (20) nach Anspruch 4, wobei die makroskopisch dreidimensionale Oberfläche einen Oberflächentopographieindex von mindestens ungefähr 0,20 aufweist.

6. Einwegwischartikel (20) nach Anspruch 4, wobei die makroskopisch dreidimensionale Oberfläche ein durchschnittliches Höhendifferenzial von mindestens ungefähr 0,5 mm aufweist.

7. Einwegwischartikel (20) nach Anspruch 4, wobei die makroskopisch dreidimensionale Oberfläche einen durchschnittlichen Abstand von Erhebung zu Erhebung von mindestens ungefähr 2,0 mm aufweist.

## Revendications

1. Article d'essuyage jetable (20), ledit article d'essuyage jetable (20) comprenant :
a) un matériau de nappe élastique (200) ; et
b) au moins une nappe non tissée élastique (100),
dans lequel ladite au moins une nappe non tissée élastique (100) est attachée à ladite nappe élastique (200) dans une relation face à face dans au moins deux zones, et selon quoi ladite au moins une nappe non tissée élastique (100) est froncée entre lesdites au moins deux zones par contraction dudit matériau de nappe élastique (200) fournissant une surface macroscopiquement tridimensionnelle de ladite au moins une nappe non tissée élastique (100) et dans laquelle à la fois ledit matériau de nappe élastique (200) et ladite au moins une nappe non tissée élastique (100) sont étirables à au moins 125 % de leur longueur relâchée et reprennent au moins 40 % de leur allongement au relâchement de la force d'allongement.

2. Article d'essuyage jetable (20) selon la revendication 1, dans lequel ledit matériau de nappe élastique (200) comprend un matériau polymère choisi dans le groupe constitué de polyoléfines telles que le polyéthylène ou le polypropylène, des élastomères de polyoléfine, des polyesters, des élastomères de polyester, du poly(butylène téréphtalate), du polyéthylène téréphtalate, de l'éthylène-acétate de vinyle ou nylon, et leurs mélanges et copolymères.

3. Article d'essuyage jetable (20) selon la revendication 1, dans lequel ledit au moins un non-tissé élastique (100) comprend des fibres qui sont naturelles ou synthétiques ou leurs mélanges.

4. Article d'essuyage jetable (20) selon la revendication 1, dans lequel ledit au moins un non-tissé élastique (100) comprend, en outre, une surface externe, macroscopiquement tridimensionnelle ayant un ordonnancement aléatoire, non répétitif de pics et de vallées, dans lequel la surface macroscopiquement tridimensionnelle a un Indice de Topographie de Surface d'au moins environ 0,15.

5. Article d'essuyage jetable (20) selon la revendication 4, dans lequel ladite surface macroscopiquement tridimensionnelle a un Indice de Topographie de Surface d'au moins environ 0,20.

6. Article d'essuyage jetable (20) selon la revendication 4, dans lequel ladite surface macroscopiquement tridimensionnelle a une Différence de Hauteur Moyenne d'au moins environ 0,5 mm.

7. Article d'essuyage jetable (20) selon la revendication 4, dans lequel ladite surface macroscopiquement tridimensionnelle a une distance moyenne de pic à pic d'au moins environ 2,0 mm.
